Europäisches Patentamt

European Patent Office

Office européen des brevets

(19)

(11) Publication number: **0 010 917**
**A1**

## (12) EUROPEAN PATENT APPLICATION

(21) Application number: **79302301.1**

(22) Date of filing: **22.10.79**

(51) Int. Cl.³: **G 01 N 33/48,** G 01 N 33/92, A 61 B 10/00, A 61 B 5/00

(30) Priority: **23.10.78 AU 6492/78**

(43) Date of publication of application: **14.05.80**
**Bulletin 80/10**

(84) Designated Contracting States: **FR GB**

(71) Applicant: **UNIVERSITY OF SYDNEY, Parramatta Road, Sydney New South Wales 2006 (AU)**

(72) Inventor: **Duck-Chong, Coral Gwenda, 92 Barons Crescent, Hunters Hill, New South Wales 2110 (AU)**

(74) Representative: **Machin, Peter Charles et al, Abel & Imray Northumberland House 303-306 High Holborn, London WC1V 7LH (GB)**

(54) **Method of assessing the foetal lung maturity.**

(57) A method of determining foetal lung maturity by reference to the lamellar body-derived phospholipid level in amniotic fluid surrounding a foetus. A sample of the amniotic fluids is subjected to centrifugation over a cushion material, and lamellar body material which forms a band at the interface between the cushion material and the amniotic fluid during centrifugation is isolated and analysed to determine its phospholipid level. A phospholipid level which is determined to be greater than about 3.5 to 5.0 mg/dl of amniotic fluid indicates a mature foetal lung.

EP 0010917 A1

-1-

DETERMINATION OF FOETAL LUNG MATURITY AND PHOSPHOLIPID LEVEL

This invention relates to a method of isolating a lamellar body fraction from amniotic fluid and to a method of assessing foetal lung maturity. Although the first of these methods is not uniquely applicable to assessment of foetal lung maturity, it is herein described in such context for convenience of reference.

Infants who are delivered before their lungs are sufficiently mature are at risk of developing respiratory distress syndrome, a potentially fatal condition. Therefore it is necessary to make an assessment of the lung maturity of a foetus before acting on a decision to reduce the normal full term of an at-risk pregnancy. Such assessment has been made for some years but, flowing from work done towards development of the present invention, it is thought that methods which have been used in the past and which are hereinafter referred to may, in some circumstances, yield results of doubtful validity.

Lamellar bodies are produced by secretory cells in the alveolar epithelium and their membranous content, which is rich in phospholipid (mainly lecithin), is secreted into the alveolar spaces to provide a major component of pulmonary surfactant. Phospholipid-rich surfactant passes from the foetal lung and into the amniotic fluid where it accumulates as gestation proceeds.

A method of isolating phospholipid of lamellar body origin from whole amniotic fluid has now been derived and, moreover, it has now been determined by empirical processes that the level of accumulated lamellar body-derived phospholipid in the amniotic fluid provides a measure of the foetal lung maturity. Thus, it has been determined that, in general, a lamellar body-derived phospholipid level greater than about 3.5 mg/dl of amniotic fluid (i.e. greater than 0.14 mg phospholipid phosphorus/dl of amniotic fluid) may be taken as an indication of a substantially mature lung, and hence a level less than about 3.5 may be taken as providing indication of a high risk of respiratory distress syndrome in a delivered infant. A lamellar body-derived phospholipid level within the range 3.5 to 5.0 mg/dl of amniotic fluid may be taken as a borderline range which indicates some risk of relatively mild respiratory problems being present in a delivered infant, but a level greater than about 5.0 may be taken as indicative of a completely mature lung.

The derived method of isolating lamellar body phospholipid forms one basis of the present invention, and the determination made of the relationship between the level of lamellar body-derived phospholipid and lung maturity forms another basis of the invention.

Before proceeding to define and describe the present invention in specific terms, a brief reference is made to the currently practised (prior art) methods of assessing foetal lung maturity using amniotic fluid. These methods, which are all documented in the relevant literature, include determination of the lecithin/ sphingomyelin ratio (the L/S ratio), the total phospholipid content and the total lecithin content, and fluorescence polarisation. Of these methods of determination, the L/S ratio is the most widely used, and all of these methods involve relatively low speed centrifugation of the amniotic fluid for the purpose of removing unwanted cell debris and whole cells.

Thus, in the prior art methods, a simple laboratory centrifuge is employed for the purpose of removing cell debris and whole cells, and not for the purpose of isolating lamellar body phospholipid from the amniotic fluid. The quantitative testing is carried out on the supernatant, which has been assumed to contain most of the lung derived phospholipid. Because the lamellar body phospholipid is not isolated, the prior art methods lack specificity, due to the possible presence of phospholipids from sources other than the lung. It has been established that, in the prior art methods, up to 70% of lung derived phospholipid may inadvertently be removed from the supernatant during centrifugation.

The present invention seeks to avoid the above problems by providing a novel process for isolating lamellar body fractions from amniotic fluid and, then, for determining the phosphorus (and thereby the phospholipid) content of the isolated fraction, whereby a measure of the lung maturity of the foetus may be determined.

Thus, the present invention provides a method of isolating lamellar body phospholipid from amniotic fluid and which comprises centrifuging the amniotic fluid over a cushion of a material that has a density sufficient to retain lamellar body phospholipid at the interface between the cushion material and the amniotic fluid during centrifugation whilst allowing whole cells and cell debris to sediment through the cushion material, and collecting lamella body material which forms a band at the interface during centrifugation.

Lamellar body phospholipid has a buoyant density in the region of 1.03 to 1.07 and the density of the cushion material must be selected so as to preclude substantial migration of the lamellar body material into the cushion material during centrifugation.

-4-

The centrifugal field strength required to achieve the desired separation of lamellar body material from cells and cell debris is variable as a function of time. Thus, an adequate isolation of lamellar body material may be achieved under the influence of a relatively low centrifugal field provided that centrifugation is maintained for a sufficiently long period of time. In the interest of reducing processing time, centrifugation is preferably effected under the influence of a high centrifugal field, i.e. one which is not less than about 8000 to 10000 g.

A centrifugal force of about 115,000 g. has been found particularly suitable in working the above defined method. This is obtainable by using an Airfuge type 350666 ultracentrifuge as marketed by Beckman Instruments International S.A. of Geneva, Switzerland. Centrifugation has been effected for 15 minutes at 25 p.s.i., using this instrument.

When employing a centrifugal force in the order of 115,000 g. a preferred cushioning material is 15% (w/v) solution of "Ficoll 70", preferably containing 0.9% NaCl. "Ficoll 70" is a registered trade mark of the company Pharmacia and it comprises a high molecular weight sucrose polymer, having a molecular weight of 70000.

The present invention further provides a method of assessing the lung maturity of a foetus and which comprises the steps of:

(a) obtaining a sample of the amniotic fluid surrounding the foetus,

(b) centrifuging the fluid over a cushion of a material that has a density sufficient to retain lamellar body phospholipid at the interface between the cushion material and the amniotic fluid during centrifugation whilst allowing whole cells and cell debris to sediment through the cushion material,

(c) collecting lamellar body material which forms a band at the interface during centrifugation, and

(d) determining the phospholipid level of the lamellar body material.

If the phospholipid level is determined to be greater than about 3.5 to 5.0 mg/dl of amniotic fluid, indication of a mature lung is obtained, as previously mentioned.

Determination of the phospholipid level of the lamellar body fraction may be performed in any one of a number of known ways, but is preferably performed in accordance with the following procedure:

Phospholipid phosphorus is first converted to inorganic phosphate by heating a dried lipid extract derived from the collected lamellar body material briefly over a Bunsen flame or an equivalent source of heat in the presence of magnesium nitrate. The resultant ash is then treated with dilute hydrochloric acid at 95°C. The resultant solution containing inorganic phosphate has added to it "Triton X-100" (Rohm and Haas) and an acidic ammonium molybdate-malachite green reagent, and its absorbance is measured at 650 nm after five minutes at room temperature. The absorbance is then compared with standard values to obtain a measure of the phospholipid phosphorus level, from which the phospholipid content may be derived, by multiplying by a factor of 25.

Thus, the present invention may be defined further as providing a method for determining the phosphorus content in a lamellar body phospholipid sample and which comprises digesting the sample to produce an inorganic phosphate - acid solution, adding a non-ionic detergent such as Triton X-100 and an acidic ammonium molybdate-malachite green reagent, measuring the absorbance of the resultant sample and comparing the obtained measurement with a standard phosphorus solution.

The invention will be more fully understood from the following described Examples of methods of working the invention.

0010917

-6-

<u>EXAMPLE I</u>

Amniotic fluid samples were obtained by amniocentesis from a number of women who were suffering complicated pregnancies and for whom a determination was to be made as to the most appropriate time for pre-normal-term delivery.

Technical grade chloroform was washed and redistilled, then stored in the dark near $0^{o}$C. All other reagents (as hereinafter referred to) were analytical grade and glass distilled water was used throughout. Glassware was washed by overnight immersion in conc. sulphuric acid containing about 5% conc. nitric acid and finally rinsed with glass distilled water.

Lamellar body fractions were isolated from each of the amniotic fluid samples and the phospholipid content was in each case determined by the following procedure:

Amniotic fluid (100 µl) was layered carefully over 75 µl of 15% (w/v) "Ficoll 70" (Pharmacia) in 0.9% NaCl in a 4.8 x 19.9 mm cellulose nitrate tube. "Ficoll 70" was used because it has a suitable density and molecular weight. A sharp interface between the layers is desirable. This was readily achieved by dispensing the samples slowly from a 100 µl syringe with the tip held against the side of the tube. Two tubes were prepared in this way for each determination. The tubes were centrifuged in the A-100 rotor of a Beckman Airfuge for 20 min. at 25 p.s.i. (115,000 $g_{av}$) at ambient temperature.

The lamellar body fraction formed a distinct band at the interface between the two layers.

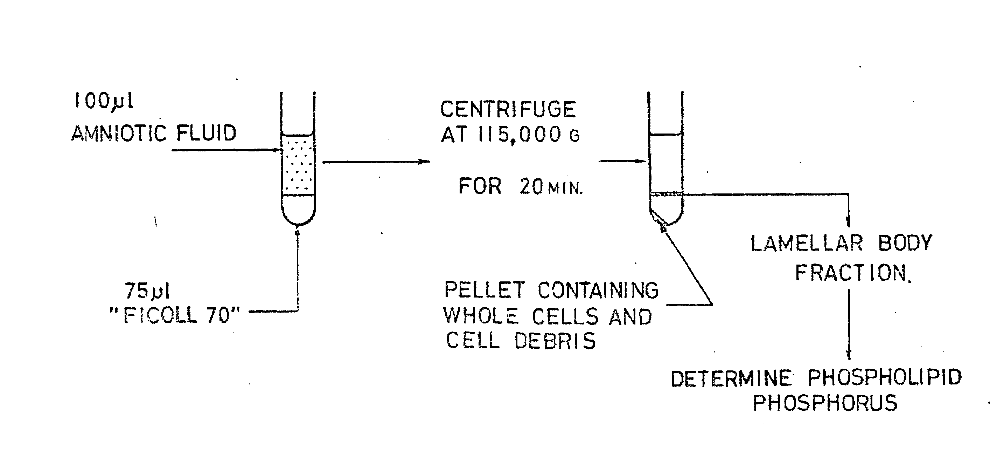

After centrifuging, the supernatant above the lamellar body fraction was carefully removed by a Pasteur pipette and discarded. The lamellar body fraction was collected in a clean Pasteur pipette, taking just sufficient of the "Ficoll" layer to ensure complete recovery of all visible material at the interface. The above steps are shown diagrammatically in the attached drawing.

The lamellar body fraction was transferred to 4 ml chloroform-methanol (2:1, v/v) in a stoppered centrifuge tube and rinsed from the Pasteur pipette by drawing the mixture up into the pipette several times.

The lamellar body fraction was collected from the second Airfuge tube in the same way using a clean Pasteur pipette and combined with the first sample in the chloroform-methanol.

The chloroform-methanol-extract was washed with 0.8 ml 0.9% NaCl and then with 0.6 ml chloroform-methanol-0.9% NaCl (3:48:47, by vol.). The samples were centrifuged gently after each wash to separate the two phases. The upper aqueous phase was removed by a Pasteur pipette, together with any white material which collected at the interface, and discarded.

The washed extract was transferred to a 17 mm test tube and evaporated to dryness in a water bath at 80-90°C. After the addition of 0.33 ml 70% perchloric acid, the samples were digested at 180°C for 45-90 min. Distilled water (2.9 ml) was added to each tube followed by 0.4ml 2.5% ammonium molybdate and then, after mixing, 0.4ml 10% ascorbic acid.

After 90 min at 37°C (or 20 min. at 50°C) the optical density was measured at 820 nm using a Turner Model 330 spectrophotometer. Standards, which were not digested, contained 0-4 µg P (as $KH_2PO_4$) and 0.3 ml perchloric acid instead of 0.33 ml. The phospholipid content was calculated by multiplying the derived phospholipid-phosphorus value by 25.

The lamellar body phospholipid content was then expressed in mg/dl of amniotic fluid and related to the 3.5 mg/dl parameter referred to previously in this specification.

Electron microscopy showed that the fraction obtained by the above described procedure did not always consist entirely of lamellar body-derived material. Thus, as well as some unidentified contaminating material, the fraction also contained some whole cells. However, it has been established that the contribution of the contaminants to the phospholipid content of the lamellar body fraction obtained was very low. In practical terms, the above described method gave a satisfactory estimate of the lamellar body phospholipid content of amniotic fluid.

Fresh samples of amniotic fluid are preferred for analysis using the above procedure. It is thought that samples to be analysed might be frozen, stored and thawed prior to analysis but such procedure is to be subjected to further analysis.

The lamellar body phospholipid content of amniotic fluid was investigated as a function of gestational age. The concentration of lamellar body phospholipid in amniotic fluid increased with gestational age, but the time of onset and the rate of the increase showed considerable variation between individuals. Eleven infants who were born of mothers from whom amniotic fluid samples were taken developed respiratory distress syndrome soon after birth, with tachypnea, chest recession, grunting, nasal flare and classical changes in chest X-ray, and required oxygen for 3 - 14 days. In all eleven cases, the measured lamellar body phospholipid value was less than 3.5mg/dl at the time of amniocentesis, 2 - 10 days before delivery. In contrast, all of two hundred and thirty-six infants delivered within 2 days of amniocentesis with values greater than 5.0mg/100ml were free from respiratory problems of this nature. The latter group included forty infants delivered before 37 weeks' gestation.

In the case of twenty-six infants from whom a measured lamellar body phospholipid value in the (borderline) range of 3.5 to 5.0 mg/dl was obtained prior to delivery, twenty-two of the infants showed no serious post-delivery respiratory problems and four of the infants revealed mild respiratory problems (not respiratory distress syndrome) which required treatment for a few days.

The contribution of phospholipid of lamellar body origin to the total phospholipid of amniotic fluid has been found to be highly variable and apparently independent of the "maturity" of the amniotic fluid, as defined by its lamellar body phospholipid content. When the data were divided into 3 groups on the basis of the lamellar body phospholipid content of the amniotic fluid, mean values for the ratio "lamellar body/total phospholipid" were 64%, 74% and 75% respectively for samples with low, medium and high lamellar body phospholipid.

.The above described procedure for determining the phospholipid content of isolated lamellar body phospholipid has yielded acceptable results, but is time-consuming. As a second example of the invention, a further method has been developed to satisfy a requirement for a more convenient, less time-consuming process.

Thus, it has been established that phospholipid digestion can be achieved in a few seconds by ashing over a Bunsen flame in the presence of magnesium nitrate. This rapid digestion procedure has been coupled with a modification of a sensitive method for determining inorganic phosphate, based on the formation of a complex between phosphomolybdate and the basic dye, malachite green. This procedure is exemplified as follows:

-10-

## EXAMPLE II

For each determination, 100 μl of amniotic fluid was layered over 75 μl of "Ficoll 70" and centrifuged in an Airfuge exactly as described in the preceding example. The supernatant above the lamellar body fraction was discarded and the lamellar body fraction was collected in a Pasteur pipette, as described in the above example, and then transferred with rinsing into 3 ml of chloroform-methanol (2:1, v/v). The chloroform-methanol extract was washed with 0.6 ml 0.9% NaCl, then 0.45 ml chloroform-methanol-0.9% NaCl (3:48:47, by vol.), by the procedure which has been been set forth in the preceding example.

The washed extract was transferred to a 17 mm test tube, mixed with 30 μl of magnesium nitrate solution (10% w/v Mg $(NO_3)_2$. 6 $H_2O$ in methanol) and evaporated to dryness. Digestion of the phospholipid was achieved by heating the sample for 15 sec. in the top of a Bunsen flame, then lowering the tube so that its base was at the tip of the blue cone of the flame and heating steadily for a further 10 sec.

After cooling to less than $100^{O}C$, 1 ml 1 M HCl was added. The tube was covered with a glass marble to minimize evaporation and heated together with suitable standards at 90-95$^{O}C$ for 5 min., then cooled to room temperature by immersion in tap water before proceeding with the determination of inorganic phosphate. Standards containing 0-0.5 μg inorganic phosphate-phosphorus $(KH_2PO_4)$ in 1 ml 1 M HCl were prepared in tubes in which 30 μl of magnesium nitrate solution had previously been evaporated to dryness and digested as described above.

Inorganic phosphate was determined by adding 30 μl 1%(w/v) "Triton X-100" to each tube (containing standard or sample in 1 ml 1 M HCl), followed by 2 ml of colour reagent.

The colour reagent was prepared freshly every 3 weeks as follows:

-11-

Solution 1:  4.2% ammonium molybdate in HCl. Conc. HCl was diluted to 4.5 M then used to dissolve the molybdate, $(NH_4)_6 Mo_7 O_{24}.4H_2O$.

Solution 2:  Malachite green (B.D.H., Product No. 34045, Colour index 42000). A 3% (w/v) solution in distilled $H_2O$ was prepared just before use.

Solution 1 (1 vol.) was mixed with Solution 2 (3 vol.) and left at room temperature for 2 - 3 hrs with occasional mixing, before filtering through Whatman No. 1 filter paper.

The contents of the tubes were mixed thoroughly following addition of the colour reagent and then left at room temperature without further mixing. The absorbance at 650 nm was determined 5 - 40 minutes after the addition of the colour reagent, using disposable plastic cuvettes. The lamellar body phospholipid content of the samples was then calculated by multiplying the derived phospholipid phosphorus value by 25, and the results were expressed in terms of mg phospholipid/dl of amniotic fluid.

-12-

CLAIMS:

1. A method of assessing the lung maturity of a foetus and which comprises the steps of;

(a) obtaining a sample of the amniotic fluid surrounding the foetus,

(b) centrifuging the fluid over a cushion of a material that has a density sufficient to retain lamellar body phospholipid at the interface between the cushion material and the amniotic fluid during centrifugation whilst allowing whole cells and cell debris to sediment through the cushion material.

(c) collecting lamellar body material which forms a band at the interface during centrifugation, and

(d) determining the phospholipid level of the lamellar body material.

2. The method as claimed in claim 1, wherein the amniotic fluid is subjected to a centrifugal force not less than 8000 g.

3. The method as claimed in claim 1, wherein the amniotic fluid is subjected to a centrifugal force in the region of 115000 g.

4. The method as claimed in claim 3, wherein the cushion material comprises a sucrose polymer having a molecular weight in the region of 70000.

-13-

5. The method as claimed in any one of claims 1 to 4, wherein the phospholipid level of the lamellar body material is determined by digesting the collected lamellar body material to produce an inorganic phosphate-acid solution and determining the phosphorus content colorimetrically by comparing the solution with a standard phosphorus solution.

6. The method as claimed in any one of claims 1 to 4, wherein the phospholipid level of the lamellar body material is determined by digesting the collected lamellar body material to produce an inorganic phosphate-acid solution, adding a non-ionic detergent and an acidic ammonium molybdate-malachite green reagent, measuring the absorbance of the resultant solution and comparing the measurement with a standard phosphorus solution.

7. A method of isolating lamellar body phospholipid from amniotic fluid and which comprises; centrifuging the amniotic fluid over a cushion of a material that has a density sufficient to retain lamellar body phospholipid at the interface between the cushion material and the amniotic fluid during centrifugation whilst allowing whole cells and cell debris to sediment through the cushion material, and collecting lamellar body material which forms a band at the interface during centrifugation.

8.   The method as claimed in claim 7, wherein the amniotic fluid is subjected to a centrifugal force not less than 8000 g.

9.   The method as claimed in claim 7, wherein the amniotic fluid is subjected to a centrifugal force in the region of 115000 g.

10. The method as claimed in claim 9, wherein the cushion material comprises a high molecular weight sucrose polymer.

11. A method of determining the phospholipid level of the lamellar body material in a sample of amniotic fluid which comprises the steps of:

(a) centrifuging the fluid over a cushion of a material that has a density sufficient to retain lamellar body .phospholipid at the interface between the cushion material and the amniotic fluid during centrifugation whilst allowing whole cells and cell debris to sediment through the cushion material.

(b) collecting lamellar body material which forms a band at the interface during centrifugation, and,

(c) determining the phospholipid level of the lamellar body material.

100µl
AMNIOTIC FLUID

CENTRIFUGE
AT 115,000 G

FOR 20 MIN.

75µl
"FICOLL 70"

PELLET CONTAINING
WHOLE CELLS AND
CELL DEBRIS

LAMELLAR BODY
FRACTION.

DETERMINE PHOSPHOLIPID
PHOSPHORUS

| | | |
|---|---|---|

**European Patent Office**

**PARTIAL EUROPEAN SEARCH REPORT**
which under Rule 45 of the European Patent Convention
shall be considered, for the purposes of subsequent
proceedings, as the European search report

Application number

EP 79302301.1

| | DOCUMENTS CONSIDERED TO BE RELEVANT | | CLASSIFICATION OF THE APPLICATION (Int. Cl.) 3 |
|---|---|---|---|
| Category | Citation of document with indication, where appropriate, of relevant passages | Relevant to claim | |
| | No relevant documents have been disclosed | | G 01 N 33/48<br>G 01 N 33/92<br>A 61 B 10/00<br>A 61 B 5/00 |
| | | | **TECHNICAL FIELDS SEARCHED (Int.Cl.) 3**<br><br>G 01 N 33/00<br>G 01 N 31/00<br>G 01 N 15/00<br>A 61 B 5/00<br>A 61 B 10/00<br>A 61 B 17/00 |

| **INCOMPLETE SEARCH** | **CATEGORY OF CITED DOCUMENTS** |
|---|---|
| The Search Division considers that the present European patent application does not comply with the provisions of the European Patent Convention to such an extent that it is not possible to carry out a meaningful search into the state of the art on the basis of some of the claims.<br>Claims searched completely: 2-11<br>Claims searched incompletely:<br>Claims not searched:<br>Reason for the limitation of the search: 1: Method for treatment of the human or animal body by surgery or therapy (See article 52(4) of the European Patent Convention). | X: particularly relevant<br>A: technological background<br>O: non-written disclosure<br>P: intermediate document<br>T: theory or principle underlying the invention<br>E: conflicting application<br>D: document cited in the application<br>L: citation for other reasons<br><br>&. member of the same patent family, corresponding document |

| Place of search | Date of completion of the search | Examiner |
|---|---|---|
| VIENNA | 10-01-1980 | LUDWIG |

EPO Form 1505.1  06.78